# EUROPEAN PATENT APPLICATION

(11) **EP 2 801 581 A1**
(43) Date of publication of application: **12.11.2014**
(21) Application number: 12821233.9
(22) Date of filing: 27.12.2012
(51) Int. Cl.: C07J 71/00, C07J 75/00, A61K 31/58, A61P 25/00

(54) **SPIROSTEROIDAL SYSTEMS HAVING NEUROACTIVE, ANTI-INFLAMMATORY EFFECTS**

(30) Priority: 27.12.2011 CU 20110244
(71) Applicant: Centro de Investigación y Desarrollo de los Medicamentos (CIDEM), La Habana 10400 (US); Garcia Pupo, Laura, San Miguel del Padron, La Habana 10600 (CU); Nuñez Figueredo, Yanier, Cotorro, La Habana 14000 (CU); Tacoronte Morales, Juan Enrique, La Habana 11300 (CU); Verdecia Reyes, Yamila., La Habana 10300 (CU); Ochoa Rodriguez, Estael, La Habana 10300 (CU)
(72) Inventor: GARCIA PUPO, Laura, San Miguel del Padron La Habana 10600 (CU); NUÑEZ FIGUEREDO, Yanier, Cotorro La Habana 14000 (CU); TACORONTE MORALES, Juan Enrique, La Habana 11300 (CU); VERDECIA REYES, Yamila., La Habana 10300 (CU); OCHOA RODRIGUEZ, Estael, La Habana 10300 (CU)
(74) Representative: Capitán García, Maria Nuria
(86) International application number: PCT/CU2012/000008
(87) International publication number: WO 2013/097835

(57) **Abstract**

This invention is related to chemistry and pharmacy and, in particular, to the production of novel molecular entities: esterane derivatives fused with spirostanes rings, acting upon the Central Nervous Systems (CNS).

From diosgenin, a naturally occurring sapogenin, with some subsequent transformations thereof, spirosteroid derivatives of the I-IV general formula can be obtained, with a cyclopentaneperhydrophenantrene nucleus fused to a 25R-spirostanes nucleus.

Such molecular entities have an anti-inflammatory and anti-glutamatergic actions that can be used to treat inflammatory, cerebrovascular, neurodegenerative, neuropsychiatric, and neurologic diseases.

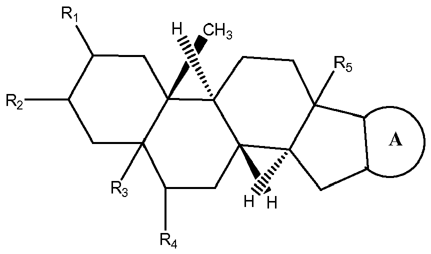

## Description

### OBJECT OF THE INVENTION

This invention is related to the chemical and pharmaceutical branches, and more specifically with obtaining new molecular entities, synthetic variants of steranes fused steroids of a general formula:

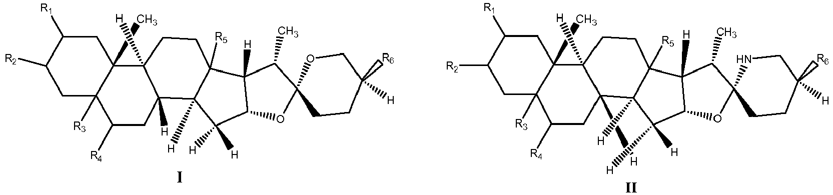

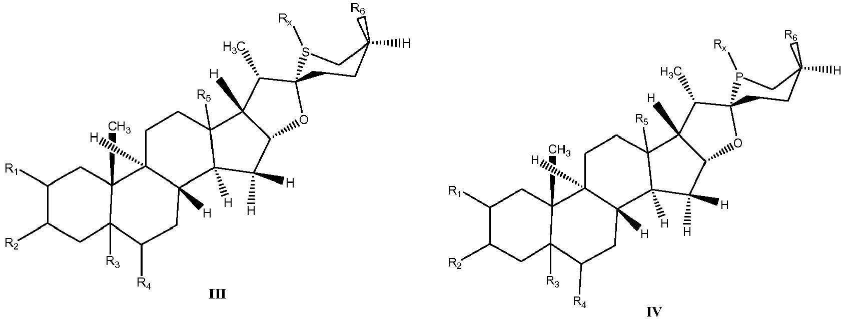

For compounds of general formula **I, II, III** and **IV,** R₁, R₂ and R₄ represents H, hydroxyl, cetal, alcoxyl, alkanyloxyl, alkenoxyl and alkoxylcarbonyloxyl groups (preferable alkyl groups having up to 8 carbon atoms such as methyl, ethyl, propyl, butyl, pentyl, hexyl, heptyl, and octyl and all chain isomers thereof).

For compounds of general formula **I, II, III** and **IV,** R₁, R₂ and R₄ also represents an amine group, preferable substituted with alkylamines, dialkylamines, alkenylamines, dialkenylamines, aminecarbonyloxyl, alkinylamines and dialkinylamines groups (preferable alkyl groups having up to 8 carbon atoms such as methyl, ethyl, propyl, butyl, pentyl, hexyl, heptyl, and octyl and all chain isomers thereof).

For compounds of general formula **I, II, III** and **IV,** R₁, R₂ and R₄ also represents amide, thiol, sulphinyl, sulphonamide and sulphonyl groups, preferable substituted with alkylaryl, alkanoyloxyaryl, alkenoyloxyaryl and alkenylaryl groups (preferable alkyl groups having up to 8 carbon atoms such as methyl, ethyl, propyl, butyl, pentyl, hexyl, heptyl, and octyl and all chain isomers thereof).

For compounds of general formula **I, II, III** and **IV,** R₁, R₂ and R₄ also represents an cyane, thiocyane, isothiocyane groups, preferable substituted with alkyl, acidalkyl, alkanyloxylalkyl, arylalkyl, heteroarylalkyl, arylalkenyl, heteroarylalkenyl, arylalkinyl, arylalkylalkinyl, alkanoyloxyarylalkylalkinyl, heteroaryloxyalkinyl, heteroaryloxyalkinyl, oxoalkinyl, or cetal groups, wherein the cyanilalkinyl substituents can be substituted in turn by heteroarylalkinyl, hydroxyalkinyl, alcoxyalkinyl, aminoalkinyl and acyloaminoalkinyl groups (preferable alkyl groups having up to 8 carbon atoms such as methyl, ethyl, propyl, butyl, pentyl, hexyl, heptyl, and octyl and all chain isomers thereof).

For compounds of general formula **I, II, III** and **IV,** R₁, R₂ and R₄ also represents a phosphoryl group, preferable substituted with alkylaryl, alkanyloxyaryl, alkenyloxyaryl and alkenylaryl groups (preferable alkyl groups having up to 8 carbon atoms such as methyl, ethyl, propyl, butyl, pentyl, hexyl, heptyl, and octyl and all chain isomers thereof).

For compounds of general formula **I, II, III** and **IV,** R₃ represents an H or hydroxyl group.

For compounds of general formula **I, II, III** and **IV,** R₅ represents H, hydroxyl, cetal, amine, thiol and cyane groups.

For compounds of general formula **I, II, III** and **IV,** R₆ represents methyl, lipid chains derived from mono or polyunsaturated fatty acids having up to 24 carbon atoms and proteins union site groups.

For compounds of general formula **III** and **IV,** Rₓ represents alkyl groups having up to 8 carbon atoms such as methyl, ethyl, propyl, butyl, pentyl, hexyl, heptyl, and octyl and all chain isomers thereof, wherein x is different from zero.

For compounds of general formula **III** and **IV,** Rₓ also represents alkylaryl, alkanyloxyaryl, alkenyloxyaryl and alkenylaryl groups, preferable alkyl groups having up to 8 carbon atoms such as methyl, ethyl, propyl, butyl, pentyl, hexyl, heptyl, and octyl and all chain isomers thereof, wherein x is different from zero.

These novel compounds can serve as a basis for therapeutic drugs to treat anxiety, ischemia, epilepsy, hypertension and other cardiovascular, cerebrovascular, neurodegenerative, neuropsychiatric, and neurological disorders, as well as other disorders related to acute and chronic inflammation.

Compounds of I, II, III and IV type are obtained by tosylation of 3β-hydroxy group and hydroxylation of 6β-one group, in presence of carbonates. Oxidative reactions can employ Jones's reactive (CrO₃-H₂SO₄), Collins's reactive (CrO₃-acetone) or pyridinium chlorocromate, as oxidant agents according to Aburatani's method; or with oxoammonium-iodine salts.

### BACKGROUND OF THE INVENTION

Steroids were one of the first pharmacological entities denominated privileged anti-inflammatory structures. Generally, most steroid molecules act as immunosuppressive agents, through molecular mechanisms including rapid non-genomic and genomic effects. However some steroids, such as hydrocortisone, cortisone, prednisone, prednisolone, fludrocortisone, deoxycorticosterone, methylprednisolone, triamcinolone, paramethasone, betamethasone, dexamethasone, triamcinolone acetonide, acetoxyprenolone, among others, causes some toxicological effects in several organs. Therefore long-term clinical usage of those molecules induces different side effects, such as cellular metabolic disorders, osteoporosis, hypertension and gastrointestinal diseases.

In other hand, the well-known neurosteroids have sedative, anesthetic and anti-convulsive affects in animals and humans. Such biological activity are due to the modulation of neuronal excitability, through the interaction between membrane receptors and ion channels, principally GABAA receptor, which acts in a bidirectional inhibitory system connected to different areas in the CNS.

Disclosed herein are new steroid compounds and compositions and their application as pharmaceuticals for the treatment of anxiety, ischemia, epilepsy, hypertension and other cardiovascular, cerebrovascular, neurodegenerative, neuropsychiatric, and neurological disorders, as well as other disorders related to acute and chronic inflammation.

After an analysis of the structure of the molecules tested and their effects on vitality of damaged PC12, as an indicator of their neuroprotective potential, the use of synthetic variants of steroids fused with spirostanes cycles for treating cerebrovascular, neurodegenerative, neuropsychiatric and neurological diseases is justified.

The novelty in this invention is obtaining a spirosteroid molecular system for potential application in the treatment of cardiovascular, cerebrovascular, neurodegenerative, neuropsychiatric and neurological diseases, as well as the possibility of obtaining these spirosteroid systems using a simple method of synthesis and steroid sapogenin-like diosgenin, hecogenin and solasodine as starting materials.

There are several patents describing steroids derivatives for treating CNS and inflammatory diseases. In such cases, however, no description is made of the fusion of these nucleuses or the inclusion of spirostanes cycles to form a new pharmacologic entity. Patents using different substituents of the spirosteroid nucleus, having no relation with the subject matter of our invention are listed below:
Patents US6909007, US186708 and US5116829 describe the process of obtaining steroid molecules as potential anti-inflammatory entities less toxic than conventional pharmaceuticals, but use hydrogen-type substituents, alkyl and alkenyl chains, and aromatic rings of the phenyl, carboxyphenyl, acetoxyphenly, methoxycarboxyphenyl and dimethylcarboxyphenyl type. Patent 5599807 describes the process of obtaining steroid molecules with an ester group between carboxyl group of kinoloncarboxylic acid and alcoholic hydroxyl group in position 21, with anti-bacterial and anti-inflammatory activities without long-term immunosuppression.

Patent US20030092692 describes chemical production and application of known and novels 7α-hydroxy steroid molecules on *in vitro* cytoprotection of neuronal cells and for treatment of acute diseases affecting CNS. However, described substituents only includes H, hydroxyl, sterified hydroxyl, alkyl, amine, alkylamine and dialkylamine groups. The patents US200330186953, US20090227551 and US201001304559 also describes the process of obtaining, and application as treatment of neurological diseases, of 3-hydroxy-7β-hydroxy and some cetonide derivatives, but their described substituents omit spirostanes cycles.

Patent US20040072806 describes only the methods, combinations and applications for treatment and prevention of neurological diseases, of natural 22R-hydroxy steroid derivatives substituted with spirost-5-en-3-ol groups.

### DESCRIPTION OF THE INVENTION

The spirosteroid synthetic variants, the subject matter of our invention, showed some kind of action upon neuronal cells. However, the degree of the action depends on the nature of the R substituent at the 3 and 6-positions and the nature of R₂ and R₄ substituents.

General experimental conditions: Fusion temperatures were determined with an Electrothermal 9100 capillary hot plate equipment. IR spectrums were registered by a Philips Analytical PU 9600 FTIR spectrophotometer in KBr tablets. The NMR 1H and NMR 13C were recorded by a Brucker ACF-250 spectrometer operating at 250.13 MHz and 62.50 MHz, respectively. All those records were determined at 26°C, with CDCl3 as solvent and TMS as internal reference. Spectral assignments in NMR were developed employing the correlating spectrum (HHCOSY y HCCOSY) for some compounds, edition DEPT technique, and comparing with spectral data of compounds reported from literature.

### EXAMPLES OF PROCEDURE

### EXAMPLE 1: Synthesis of 6β-hydroxy 6-one poly-hydroxy derivatives

Those compounds with 3α,5-cyclo-6-one structure, were obtained through Aburatani's method, which employs the 5-en-3β-ol system as starting material and consists in three principals stages. These experimental stages are performed in a continuous way and are the following:
- Tosylation of diosgenin/hecogenin.
- Mesylation with MsCl, triethylamine and butanone.
- Isomerization with NaHCO₃ aqueous solution to form the i-steroid.
- Oxidation of C-6 hydroxyl group with Jones's reactive, to obtain the 3α,5-cyclo-6-one derivative.
- Isomerization of i-systems to Δ² spirostanic systems in presence of Li₂CO₂/CaCO₃/DMFA/HMFTA.
- Dihydroxylation in catalytic conditions (CHCl₃/NaOH/cetyltrimethylammonium bromide), and catalysis by solid bentonite acids and pyritic ashes.
- Catalytic esterification with niobyl-vanadyl acetate/acetic anhydride.
- Epoxide opening with bentonite-pyritic ashes and a reflux in water.

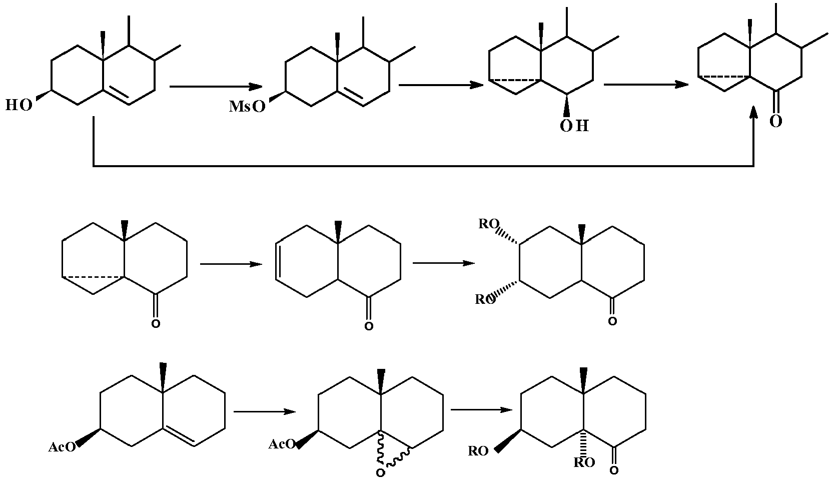

### EXAMPLE 2: Chemical and structural

### characterization of 6β-hydroxy 6-one poly-hydroxy derivatives

The spectroscopic reported data corroborates the proposed structures. In FTIR spectrums are observed the typical bands in 1350 to 850 cm⁻¹ zones, due to vibrations of spiroketal systems, associated to narrowing in C-C and C-O bonds. These evidences demonstrate there were not structural degradation changes in E and F rings. Moreover, the band in 880-910 cm⁻¹ is about twice more intense than the band in 925-910 cm⁻¹, therefore all synthesized compounds belongs to 25R series. The frequency zone 1713-1735 cm⁻¹, corresponding to acetoxy group CH₃-CO-O-, is typical for synthesized acetoxy derivatives. The band in 1360 and 1170-1175 cm⁻¹, corresponding to asymmetric and symmetric vibrations of SO₂ group (v SO₂ s and as.), are characteristic for the tosyl derivative. To this grouping corresponds the band in 1588 cm⁻¹ assignable to an aromatic v C=C.

The NMR-¹H and ¹³C spectroscopy were informative in order to elucidate the structure of synthesized compounds. Among common significant signals are those of protons from methyl groups (CH₃) 18, 19, 21 and 27, and those of protons H₂-26 and H-16α. The H₂-26 signal is complex and corresponds to both chemical shifts H-26α and H-26β, which practically does not vary in the series of studied compound. The axial H-26α is a triplet with δ = 3,32 ppm; and its multiplicity results from a germinal quasi degenerate and vecinal axial-axial (J²≅ J³= 10,6 Hz) double coupling. The H-26β signal is a double doublet with δ =3,5 ppm; and its multiplicity results from a germinal coupling (J² = 10,6 Hz) and vecinal equatorial-axial (J³ₑₐ = 2,6 Hz). With a chemical shift of δ = 4,4 ppm, the appearance of a double doublet is typical for H-16α, due to its coupling with protons 15β (equatorial), 15α and 17β (quasi-equatorials).

The signals of olefinic protons H-2 and H-3 with δ = 5,5 and 5,7 ppm, respectively, are characteristics for Δ²-6-oxo steroidal compounds. The signals of 7β and 5α protons appear superpose in 2,3-2,4 ppm. In NMR-¹³C C-2 and C-3 signals are unshielded to δ = 124,4 and 124,5 ppm, respectively, while C-4 shields to 21,7 ppm.

**Table 1. Chemical shifts (δ, ppm) of carbon atoms from A and B rings, and C-19 of (25R)-3α,5-cyclo-spirostan-6β-ol (G1) and (25R)-2,3-dihydroxy-spirostan-6β-ona (G2).**

| | **Compounds** | |
|---|---|---|
| **Carbons** # | **G1** | **G2** |
| 1 | 33.2 | 40.15 |
| 2 | 24.9 | 68.23 |
| 3 | - | 68.31 |
| 4 | 11.8 | 26.34 |
| 5 | 38.8 | 50.73 |
| 6 | 73.5 | 211.79 |
| 7 | 37.4 | 46.81 |
| 8 | 29.5 | 37.14 |
| 9 | 24.2 | 53.72 |
| 10 | 43.0 | 42.51 |
| 19 | 20.2 | 13.63 |

### EXAMPLE 2: Effects of the synthetic spirosteroids series on cellular vitality of PC12 exposed or not to glutamatergic damage

PC12 constitute a neuron-like cell line widely employed in several models of neurological diseases. The patophysiological mechanisms which can be simulated in those *in vitro* models, allows the use of PC12 cells in evaluation of therapeutic candidates.

The synthetic spirosteroids series (10 µM of each molecule) was evaluated on PC12 cells exposed to L-glutamate (20mM) for 24h. Previously, those cells were routinely cultivated in RPMI 1640 medium, supplemented with equine serum (10%) and fetal bovine serum (5%) in a 37°C and 5% CO₂ atmosphere. In the beginning of the experiment, cells were seeded in 96-well microplates at a cellular density of 150,000 cells/mL. After 24h of exposition to glutamatergic damage and spirosteroids treatment, cellular vitality was determined with 3-(4,5-dimethylthiazol-2-yl)-2,5-diphenyltetrazolium bromide (MTT), which is metabolized by mitochondrial deshydrogenases in living cells.

Our results (Table 2) demonstrate an anti-glutamatergic and neuroprotector effect of several spirosteroids molecules (S) related to some structural features. Specifically, between the most active molecules was 2α,3α-dyhydroxy-(25R)-spirostan-6-one and 3,6-dione-(25R)-spirostan. All molecules of the synthetic spirosteroids series were non cytotoxic at the concentration employed for the *in vitro* anti-glutamatergic assay (data no shown).

**Table 2. Anti-glutamatergic effect of the synthetic spirosteroids series.**

| Experimental Groups | Absorbance (540nm) | | | | | |
|---|---|---|---|---|---|---|
| Untreated | 0,557 | 0,471 | 0,519 | 0,425 | 0, 649 | 0,716 |
| L-glutamate | 0,342 | 0,341 | 0,347 | 0,534 | 0,271 | 0,385 |
| S1 | 0,619 | 0,41 | 0,396 | 0,473 | 0,527 | 0,557 |
| S2 | 0,562 | 0,372 | 0,343 | 0,42 | 0,452 | 0,536 |
| S3 | 0,538 | 0,543 | 0,357 | 0,524 | 0,505 | 0,466 |
| S4 | 0,653 | 0,605 | 0,489 | 0,607 | 0,484 | 0,643 |
| S5 | 0,646 | 0,632 | 0,378 | 0,557 | 0,543 | 0,552 |
| S6 | 0,58 | 0,592 | 0,545 | 0,562 | 0,435 | 0,589 |
| S7 | 0,516 | 0,558 | 0,437 | 0,453 | 0,391 | 0,636 |
| S8 | 0,467 | 0,28 | 0,311 | 0,547 | 0,448 | 0,482 |
| S9 | 0,412 | 0,311 | 0,27 | 0,499 | 0,501 | 0,545 |
| S10 | 0,301 | 0,257 | 0,262 | 0,499 | 0,513 | 0,41 |
| S11 | 0,364 | 0,481 | 0,392 | 0,511 | 0,47 | 0,445 |
| S12 | 0,384 | 0,423 | 0,58 | 0,501 | 0,625 | 0,486 |
| S13 | 0,571 | 0,403 | 0,593 | 0,469 | 0,626 | 0,382 |
| S14 | 0,415 | 0,381 | 0,4 | 0,586 | 0,657 | 0,659 |
| S15 | 0,423 | 0,417 | 0,579 | 0,567 | 0,612 | 0,552 |
| S16 | 0,348 | 0,394 | 0,438 | 0,53 | 0,567 | 0,563 |
| S17 | 0,345 | 0,468 | 0,329 | 0,438 | 0,545 | 0,318 |
| S18 | 0,284 | 0,346 | 0,346 | 0,44 | 0,411 | 0,464 |

## Claims

1. Spirosteroidal systems with neuroactive and anti-inflammatory effects derived from diosgenin, hecogenin and solasodine, and with spirostanic rings fused to its structures, to be used as a drug in medicine, of a general formula 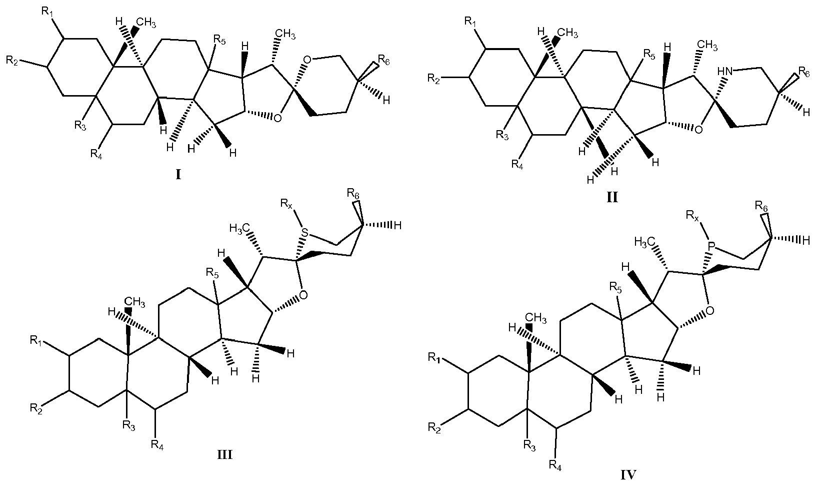 in all of its naturals conformations and configurations, wherein for compounds of the general formula I, II, III and IV, For compounds of general formula I, II, III and IV, R₁, R₂ and R₄ represents H, hydroxyl, cetal, alcoxyl, alkanyloxyl, alkenoxyl and alkoxylcarbonyloxyl groups (preferable alkyl groups having up to 8 carbon atoms such as methyl, ethyl, propyl, butyl, pentyl, hexyl, heptyl, and octyl and all chain isomers thereof).
For compounds of general formula **I, II, III** and **IV,** R₁, R₂ and R₄ also represents an amine group, preferable substituted with alkylamines, dialkylamines, alkenylamines, dialkenylamines, aminecarbonyloxyl, alkinylamines and dialkinylamines groups (preferable alkyl groups having up to 8 carbon atoms such as methyl, ethyl, propyl, butyl, pentyl, hexyl, heptyl, and octyl and all chain isomers thereof).
For compounds of general formula **I, II, III** and **IV,** R₁, R₂ and R₄ also represents amide, thiol, sulphinyl, sulphonamide and sulphonyl groups, preferable substituted with alkylaryl, alkanoyloxyaryl, alkenoyloxyaryl and alkenylaryl groups (preferable alkyl groups having up to 8 carbon atoms such as methyl, ethyl, propyl, butyl, pentyl, hexyl, heptyl, and octyl and all chain isomers thereof).
For compounds of general formula **I, II, III** and **IV,** R₁, R₂ and R₄ also represents an cyane, thiocyane, isothiocyane groups, preferable substituted with alkyl, acidalkyl, alkanyloxylalkyl, arylalkyl, heteroarylalkyl, arylalkenyl, heteroarylalkenyl, arylalkinyl, arylalkylalkinyl, alkanoyloxyarylalkylalkinyl, heteroaryloxyalkinyl, heteroaryloxyalkinyl, oxoalkinyl, or cetal groups, wherein the cyanilalkinyl substituents can be substituted in turn by heteroarylalkinyl, hydroxyalkinyl, alcoxyalkinyl, aminoalkinyl and acyloaminoalkinyl groups (preferable alkyl groups having up to 8 carbon atoms such as methyl, ethyl, propyl, butyl, pentyl, hexyl, heptyl, and octyl and all chain isomers thereof).
For compounds of general formula **I, II, III** and **IV,** R₁, R₂ and R₄ also represents a phosphoryl group, preferable substituted with alkylaryl, alkanyloxyaryl, alkenyloxyaryl and alkenylaryl groups (preferable alkyl groups having up to 8 carbon atoms such as methyl, ethyl, propyl, butyl, pentyl, hexyl, heptyl, and octyl and all chain isomers thereof).
For compounds of general formula **I, II, III** and **IV,** R₃ represents an H or hydroxyl group.
For compounds of general formula **I, II, III** and **IV,** R₅ represents H, hydroxyl, cetal, amine, thiol and cyane groups.
For compounds of general formula **I, II, III** and **IV,** R₆ represents methyl, lipid chains derived from mono or polyunsaturated fatty acids having up to 24 carbon atoms and proteins union site groups.
For compounds of general formula **III** and **IV,** Rₓ represents alkyl groups having up to 8 carbon atoms such as methyl, ethyl, propyl, butyl, pentyl, hexyl, heptyl, and octyl and all chain isomers thereof, wherein x is different from zero.
For compounds of general formula **III** and **IV,** Rₓ also represents alkylaryl, alkanyloxyaryl, alkenyloxyaryl and alkenylaryl groups, preferable alkyl groups having up to 8 carbon atoms such as methyl, ethyl, propyl, butyl, pentyl, hexyl, heptyl, and octyl and all chain isomers thereof, wherein x is different from zero.

2. Spirosteroidal systems with neuroactive and anti-inflammatory effects, of a general formula **I, II, III** and **IV** according to claim 1, where derivatives object of the present invention are obtained by synthetic methods.

3. Spirosteroidal systems with neuroactive and anti-inflammatory effects, of a general formula **I, II, III** and **IV** according to claim 1, where synthetic derivatives are obtained by acetoxylation-tosylation-mesylation catalytic processes, catalytic epoxidation, catalytic opening of epoxides, doble bond generation in the A ring of spirosteroidal system, and subsequent dihydroxylation in phase transfer mediated catalysis conditions.

4. Pharmaceutical compositions which contains as active ingredient spirosteroidal systems of general formula **I, II, III** and **IV** according to claim 1 and 2, in liquid form for oral administration, characterized as contains the active substance in 1-83%, sodium carboxymethylcellulose (0.7%) or hydroxypropylmethylcellulose (0.5%), sodium saccharine (0.4%), propylenglycol (5%), 70% sorbitol (10%), a flavor (0.1%) and water as dissolvent.

5. Pharmaceutical compositions which contains as active ingredient spirosteroidal systems of general formula **I, II, III** and **IV** according to claim 1 and 2, in capsules form for oral administration, characterized as contains the active substance in 1-40%, colloidal silicon dioxide (1%), microcrystalline cellulose (58%), magnesium stereate (0.8%), enclosed in hard gelatin capsules or hydroxypropylmethylcellulose.

6. Pharmaceutical compositions which contains as active ingredient spirosteroidal systems of general formula **I, II, III** and **IV** according to claim 1 and 2, in tablets form or granulates for oral administration, characterized as contains the active substance in 1-70%, colloidal silicon dioxide (1%), microcrystalline cellulose (20%), lactose (10%) for direct compression and magnesium stereate (0.8%).

7. Pharmaceutical compositions which contains as active ingredient spirosteroidal systems of general formula **I, II, III** and **IV** according to claim 1 and 2, in liquid form for parenteral administration, characterized as contains the active substance in 0.1-99%, sodium chloride (0.6%), sodium monobasic phosphate and potassium dibasic phosphate as pH regulators and water for injection.

8. Pharmaceutical compositions which contains as active ingredient spirosteroidal systems of general formula **I, II, III** and **IV** according to claim 1 and 2, in liquid form for nasal administration, characterized as contains the active substance in 0.1-97%, sodium chloride (0.6%), dextran 70 (1%), Carbopol 974 (0.5%), sodium monobasic phosphate and potassium dibasic phosphate as pH regulators and water for injection.

9. Pharmaceutical compositions which contains as active ingredient spirosteroidal systems of general formula **I, II, III** and **IV** according to claim 1 and 2, in sustained-release tablets form for oral administration, characterized as contains the active substance in Eudragit S 100 microencapsulated form, in 1-70%, colloidal silicon dioxide (1%), microcrystalline cellulose (20%), lactose (10%) for direct compression and magnesium stereate (0.8%).

10. Pharmaceutical compositions which contains as active ingredient spirosteroidal systems of general formula **I, II, III** and **IV** according to claim 1 and 2, in sustained-release liquid form for parenteral administration, characterized as contains the active substance in polylactic coglycolic acid microencapsulated form in 0.1-99%, sodium chloride (0.6%), sodium monobasic phosphate and potassium dibasic phosphate as pH regulators and water for injection.
